# EUROPÄISCHE PATENTSCHRIFT

(11) **EP 0 671 154 B1**
(45) Veröffentlichungstag und Bekanntmachung des Hinweises auf die Patenterteilung: **28.05.1997**
(21) Anmeldenummer: 95101644.3
(22) Anmeldetag: 07.02.1995
(51) Int. Cl.: A61F 13/15, A61F 13/46

(54) **Verfahren zur Herstellung eines saugfähigen Verbundwerkstoffs**
A Method for the manufacture of an absorbent article
Procédé de fabrication d articles absorbants

(30) Priorität: 15.02.1994 DE 9402463 U
(43) Veröffentlichungstag der Anmeldung: 13.09.1995
(73) Patentinhaber: Wolff-Dörrenberg, Beatrix, 82211 Herrsching/Ammersee (DE)
(72) Erfinder: Demhartner, Rudolf, D-80804 München (DE)
(74) Vertreter: Baronetzky, Klaus, Dipl.-Ing.

(56) Entgegenhaltungen:
- EP-A- 0 033 235
- EP-A- 0 401 189
- EP-A- 0 562 846
- EP-A- 0 576 738
- EP-A- 0 631 768
- WO-A-92/11831
- WO-A-93/22998
- GB-A- 2 004 201
- GB-A- 2 078 527
- US-A- 4 156 664
- US-A- 4 297 410
- US-A- 4 551 191
- US-A- 5 160 331

## Beschreibung

Die Erfindung betrifft ein Verfahren, gemäß dem Oberbegriff von Anspruch 1. Ein solches Verfahren ist z.B. aus der US-A-5 160 331 bekannt.

Es sind zahlreiche Versuche bekannt geworden, einen saugfähigen Verbundwerkstoff herzustellen, der einerseits nach außen hin dicht ist, andererseits jedoch auf der Innenseite auch nach Benetzung einen trockenen Eindruck vermittelt. Um dieses Ziel zu erreichen, hat man Saugkörper, beispielsweise aus Polyacrylat, bereitgestellt, die bei Befeuchtung mit Wasser, Urin oder sonstigen wässrigen Lösungen, beispielsweise auch Blut, unter Gelbildung aufquellen und eine insofern hohe Absorptionsfähigkeit aufweisen. Hierzu wird ein Superabsorber-Granulat eingesetzt, wozu beispielsweise auf die DE-A1-42 07 465 zu verweisen ist. Granulate sind gegenüber kompakten Quellkörpern zu bevorzugen, nachdem die Quellfähigkeit - vor allem bei kleiner Korngröße - aufgrund der vergrößerten Oberfläche besser ist. Um die Quellfähigkeit und damit die Absorptionsfähigkeit zu verbessern, ist es auch bekannt geworden, nahe einer feuchtigkeitsundurchlässigen Schicht möglichst kleine Korngrößen und auf der dem Körper zugewandten Seite eher größere Korngrößen zu verwenden.

Gerade Granulat kleiner Körnung neigt jedoch dazu, sich in unerwünschter Weise zu verlagern. Wenn Windeln, Krankenunterlagen usw. in kompakt zusammengepreßter Form aufbewahrt würden, wäre diese Gefahr relativ gering. Gerade bei Windeln ist jedoch ein gewisser "Flausch", also ein weicher Charakter der Körperauflage erwünscht, so daß häufig eine wenig komprimierte Aufbewahrung bevorzugt wird. Beim Transport, aber auch bei der Benutzung können sich die Granulatkörner dementsprechend verlagern.

Um dieser Gefahr zu begegenen, ist es bekannt geworden, die Granulatkörper innerhalb eines kompakten Saugkörpers vorzusehen, wozu beispielsweise auf die DE-A1-37 38 601 zu verweisen ist, die den Saugkörper von einer 0,5 bis 10 mm dicken Schicht von Zellstoffflocken und diese Zellstoffflocken von einer Vliesstoffschicht umhüllt zeigt.

Eine ungleichförmige Verteilung der Granulatkörper läßt sich mit dieser Lösung jedoch nicht verhindern.

Granulatkörper sind herstellungsbedingt als relativ scharfkantige Körner ausgebildet. Im Hinblick auf möglichst große Oberfläche ist eine eher zackige Kornstruktur sogar erwünscht. Die Sperrschicht, mit der eine Flüssigkeitsabdichtung nach außen sichergestellt wird, ist demgemäß bei granulathaltigen Verbundwerkstoffen relativ dick, allein schon, um eine Verletzung der Sperrschicht durch die Granulatkörner zu verhindern. Andererseits ist eine relativ dicke Sperrschicht oder Außenhaut ungünstig, nicht nur, was das Transport- und Benutzungsgewicht, sondern auch, was die Entsorgung angeht.

Dieser Effekt ist besonders bei Krankenunterlagen und Verbundwerkstoffen zur Inkontinenzvorsorge relevant, nachdem die Körner durch die Bewegung und das Gewicht des Patienten, die auf die Krankenunterlagen einwirken, vor der Befeuchtung geradezu wie Schmirgelkörner wirken.

Ferner ist aus der US-Patentschrift 51 60 331 ein Verfahren bekannt, bei welchem ein sogenannter Kaltleim HL 9415 im nassen Zustand klebrig bleiben und sich bei Beaufschlagung mit heißem Wasser lösen soll. Dieser Kaltleim soll für die Klebeschichten 16 und 22 verwendet werden, wobei auf diese Schichten eine Schicht saugfähigen Materials aufgebracht werden soll.

Für die Verwendung von Superabsorbern ist eine derartige Verbindung nicht geeignet.

Demgegenüber liegt der Erfindung die Aufgabe zugrunde, einen saugfähigen Verbundwerkstoff gemäß dem Oberbegriff von Anspruch 1 zu schaffen, der eine vergleichsweise dünne Außenhaut ermöglicht, wobei dennoch eine hervorragende Aufnahmekapazität für Flüssigkeiten trotz Unempfindlichkeit auch bei wechselnden Druckbelastungen des Verbundwerkstoffs bestehen soll.

Diese Aufgabe wird erfindungsgemäß durch Anspruch 1 gelöst. Vorteilhafte Weiterbildungen ergeben sich aus den Unteransprüchen.

Überraschend läßt sich dadurch, daß die Tragschicht mit den feinkörnigen Granulaten intensiv verbunden wird, die Verletzungsgefahr der Außenhaut auch dann drastisch reduzieren, wenn eine ganz dünne Sperrschicht oder Außenhaut verwendet wird. Durch das Aufkleben mittels eines feuchtigkeitsreduzierten Sprühleims werden je die untersten Granulatkörner auf der Tragschicht fixiert, so daß durch sie eine Verletzung der Außenhaut ausgeschlossen ist. Bei entsprechend hoher Dichte dieser untersten Granulatkörner wird zudem verhindert, daß die nächstliegenden Granulatkörner überhaupt noch mit der Außenhaut in Berührung kommen können. Überraschend läßt sich so trotz relativ wenig Sprühleim auch bei intensivem Hin- und Herwälzen des Patienten eine Penetration der Außenhaut ausschließen.

Es versteht sich, daß bei Bedarf weitere Schichten des Verbundwerkstoffs in entsprechender Weise ausgestattet sein können. Als Sprühleim kann ein Hot-Melt-Klebstoff verwendet werden, der mit dem hochsaugfähigen Material kompatibel ist, dieses jedoch nicht zu stark benetzen soll, um die Feuchtigkeitsaufnahmefähigkeit des Acrylats nicht zu beeinträchtigen.

Es versteht sich ferner, daß anstelle der hier bevorzugt herausgestellten Polyacrylate mit Hydroxyl-Gruppen andere hochsaugfähige Materialien sinngemäß eingesetzt werden können.

Besonders günstig ist es, daß erfindungsgemäß auch stark zerklüftete und scharfkantige Granulatkörper verwendet werden können. Durch den Sprühleim wird der jeweilige Granulatkörper von der Außenseite, die ohnehin von der eindringenden Feuchtigkeit abgewandt ist, sanft umhüllt, so daß die Ritzfähigkeit gegenüber der Außenhaut dort stark reduziert ist. Beispielsweise kann die Tragschicht, die bereits mit den hochsaugfähigen Materialkörnern versehen ist, von außen mit feuchtigkeitsreduziertem Sprühleim versehen werden, und unmittelbar hieran anschließend kann die Außenhaut aufgebracht werden, so daß sich die gewünschte intensive Verbindung ergibt, die ggf. durch kurzzeitigen Druck verbessert werden kann. Gemäß einer anderen Ausführungsform der Erfindung wird die Außenhaut mit Sprühleim versehen und dann die Tragschicht und die hochsaugfähigen Körner dort aufgebracht. Hierdurch ergibt sich auch eine Verklebung zwischen Außenhaut und Tragschicht.

Es ist auch möglich, sowohl eine vliesförmige Tragschicht als auch die Außenhaut als Tragschicht wirken zu lassen, wobei es sich versteht, daß durch die intensive Aufbringung des Acrylat-Granulats eine Art Schutzschicht für die Außenhaut entsteht.

Erfindungsgemäß besonders günstig ist es, daß durch die Konzentration hochsaugfähigen Materials nahe der Deckschicht besonders gute Absorptionsfähigkeiten und eine trockene Oberfläche der Windel oder der Inkontinenzunterlage erzielbar sind. Hierzu empfiehlt sich insbesondere ein zweischichtiger Aufbau des erfindungsgemäßen Verbundwerkstoffs mit einer geringeren Korngröße nahe der Außenhaut und einer größeren Korngröße nahe der Abdeckschicht. Der erfindungsgemäße Verbundwerkstoff erlaubt mit dieser Ausgestaltung eine Saugfähigkeit, die von oben lediglich noch eine ganz geringe Restfeuchtigkeit spüren läßt.

Gemäß einer weiteren bevorzugten Ausgestaltung der Erfindung kann der Verbundwerkstoff durch eine Abdeckbahn aus Vlieswerkstoff mit einer hydrophoben Innenseite abgedeckt sein. Durch diese Abdeckbahn wird ein rascher Eintritt der Flüssigkeit in den Verbundwerkstoff sichergestellt. Nach Eintritt der Flüssigkeit wird diese durch die wasserabstossende Wirkung der hydrophoben Innenseite wirksam daran gehindert, wieder nach außen zu treten.

Weitere Vorteile, Einzelheiten und Merkmale ergeben sich aus der nachstehenden Beschreibung mehrerer Ausführungsbeispiele anhand der Zeichnung.

Es zeigen:
- Fig. 1: eine Draufsicht auf eine Ausgestaltung eines erfindungsgemäßen saugfähigen Verbundwerkstoffs, in Form einer Inkontinenzunterlage;
- Fig. 2: einen schematischen Schnitt entlang der Line II-II aus Fig. 1;
- Fig. 3: Schnitte durch weitere Ausführungsformen eines erfindungsgemäßen saugfähigen Verbundwerkstoffs; und
- Fig. 4: eine vergrößerte Ansicht aus Fig. 3.

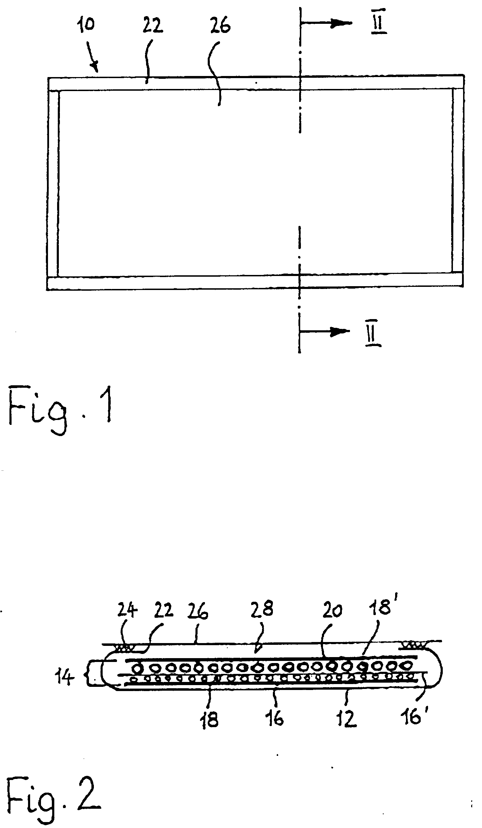

In Fig. 1 ist eine Inkontinenzunterlage 10 dargestellt, deren nach unten weisende Außenhaut als Kunststoffolie ausgebildet ist. Die Kunststoffolie kann atmungsaktiv und/oder geprägt sein, um der Außenhaut einen gewebeartigen Charakter zu vermitteln, soll jedenfalls Feuchtigkeit nicht durchlassen. Beispielsweise besteht die Außenhaut aus Polypropylen.

Der saugfähige Verbundwerkstoff 14 weist ferner - wie aus Fig. 2 ersichtlich ist - eine untere Tragschicht 16 auf, auf der eine Schicht 18 aus Acrylat-Granulat aufgebracht ist. Damit das Granulat der Schicht 18 sicher und rutschfest auf der Tragschicht 16 haftet, ist es mittels Sprühleims sehr geringer Feuchtigkeit auf der Tragschicht fixiert. Mit der geringen Feuchtigkeit des Sprühleims wird sichergestellt, daß das Granulat nicht bereits beim Aufsprühen quillt und Feuchtigkeit aufnimmt. Um dem Sprühleim eine sehr geringe Feuchtigkeit zu verleihen, wird dieser nach dem üblichen Aufschmelzen über Druckluft auf die Tragschicht 16 gesprüht. Die Druckluft wird zuvor über ein Trocknungsmittel wie Silicagel geleitet und wird dadurch so trocken, daß nur noch eine sehr geringe Restfeuchte von weniger als 0,5 % verbleibt.

In Verbindung mit getrockneter Druckluft kann auch ein aufgesprühter Hot-Melt-Klebstoff verwendet werden, wobei es sich versteht, daß dieser sowohl zu der Tragschicht als auch zu dem Acrylat kompatibel sein muß.

Als Tragschicht 16 kann ein Vlieswerkstoff, beispielsweise mit einem Gewicht von 24 g/m², oder ein als Tissue bekanntes feines und hochsaugfähiges Papier, das naßreißfest ist, verwendet werden.

Es versteht sich, daß anstelle von mit Urin reagierenden Substanzen auch andere Substanzen für die Tragschicht verwendet werden können, beispielsweise aus einem Verbund von Polypropylen und Viskose, oder vollständig aus Polypropylen. Damit wird die Geruchsbelästigung durch den saugfähigen Verbundwerkstoff nach Urinbenetzung noch weiter reduziert.

In dem dargestellten Ausführungsbeispiel ist auf der Granulatkörnerschicht 18 eine weitere Tragschicht 16' angeordnet, die etwas leichter als die untere Tragschicht 16 ausgebildet ist. Beispielsweise kann ein Vlieswerkstoff mit einem m²-Gewicht von 18 g eingesetzt werden. Auf dieser Tragschicht 16' ist mittels Sprühleim eine weitere Schicht 18' aus Acrylat-Granulat fixiert, das eine etwas größere Korngröße und damit eine relativ geringere Oberfläche aufweist. Es versteht sich, daß der Sprühleim dort so aufgebracht ist, daß er das Eindringen von Feuchtigkeit in die Tragschicht 16' nicht beeinträchtigt.

Die zweite Schicht 18' aus hochsaugfähigem Granulat ist durch eine Deckschicht 20 abgedeckt, beispielsweise aus Tissue oder Vlies mit einem m²-Gewicht von 24 g.

Zusammen mit einer Abdeckbahn 26, die auf der Deckschicht 20 angebracht sein kann, bilden die beiden Tragschichten 16 und 16', die Körnerschichten 18 und 18' sowie die Außenhaut 12 einen Superabsorber-Verbundwerkstoff 14. Dieser kann als Halbfabrikat vielfältige Anwendung finden, beispielsweise für die Herstellung von Windeln, Damenbinden, Inkontinenzunterlagen oder Betteinlagen.

In Fig. 1 ist beispielhaft eine derartige Inkontinenzunterlage dargestellt. Wie aus Fig. 2 ersichtlich ist, können die Längsränder 22 der Außenhaut 12 nach oben und innen umgelegt sein, worauf über Leimrauten 24 oder Heißprägung eine Abdeckbahn 26 aufgeklebt ist. Die Innenseite 28 der Abdeckbahn 26 hat hydrophobe Eigenschaften. Als Material für eine derartige Abdeckbahn eignet sich ein Vlieswerkstoff von etwa 15 g/m².

Der für die Tragschichten 16, 16', die Deckschicht 20 und die Abdeckbahn 26 verwendete Vlieswerkstoff besteht vorzugsweise aus einem Verbund von etwa 80 % Polypropylen und 20 % Viskose, kann aber auch bis zu 100 %, also vollständig aus Polypropylen bestehen.

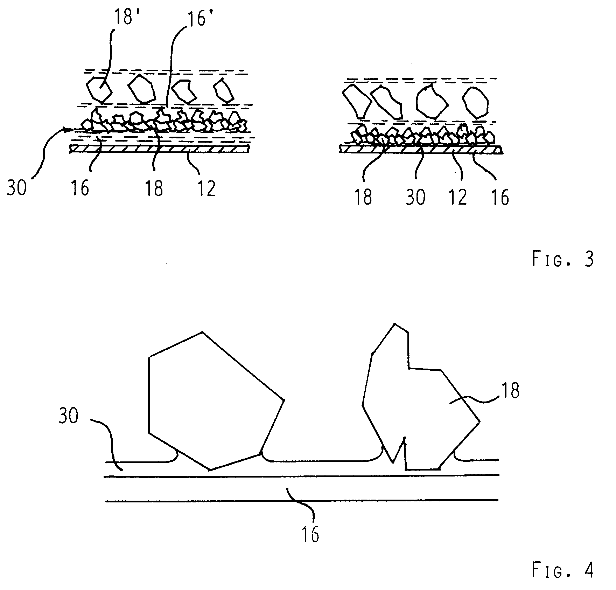

Zwei weitere Ausführungsformen eines erfindungsgemäßen Verbundwerkstoffs sind aus Fig. 3 ersichtlich. Der in Fig. 3 links dargestellte Teil, der die eine Ausführungsform wiedergeben soll, zeigt, wie Sprühleim 30 die unterste Schicht 18 der Granulatkörper einbettet und zugleich die Tragschicht 16 schützt. Unterhalb der Tragschicht erstreckt sich dann die Außenhaut 12. Oberhalb der Granulatkörperschicht 18 befindliche Granulatkörner geraten so nicht in Kontakt mit der Tragschicht 16 und damit auch nicht mit der Außenhaut 12, während sich oberhalb dieser eine weitere Tragschicht 16' und eine weitere Granulatschicht 18' erstreckt.

Das in Fig. 3 rechts dargestellte Ausführungsbeispiel zeigt die Außenhaut 12 als Tragschicht 16, wobei der Sprühleim 30 hier eine sichere Einbettung der äußersten Granulatkörperschicht 18 gewährleistet. Im übrigen entspricht diese Ausgestaltung der in der gleichen Figur links dargestellten.

Aus Fig. 4 ist ersichtlich, daß durchaus auch scharfkantige Acrylatkörner verwendet werden können, ohne daß eine Verletzung der Außenhaut 12 oder der Tragschicht 16 zu befürchten wäre. Der Sprühleim 30 paßt sich der Kontur der Granulatkörper 18 an und bettet diese ein, so daß eine harmonische Verbindung besteht.

## Patentansprüche

1. Verfahren zur Herstellung eines saugfähigen Verbundwerkstoffs, insbesondere für Windeln, Betteinlagen, Damenbinden, Inkontinenz- und Krankenunterlagen oder dgl., mit einer Tragschicht (16), auf der eine Schicht saugfähigen Materials aufgebracht wird, dadurch **gekennzeichnet,** daß mindestens ein körnig-hochsaugfähiger Teil (18) des saugfähigen Materials auf der Tragschicht (16) über eine mittels getrockneter Druckluft aufgesprühte Klebstoffschicht aufgeklebt wird.

2. Verfahren nach Anspruch 1, dadurch gekennzeichnet, daß der auf das hochsaugfähige, insbesondere aus Acrylat-Granulat bestehende oder biologisch abbaubare Material (18) aufgebrachte Sprühleim von außen aufgebracht wird.

3. Verfahren nach Anspruch 1 oder 2, dadurch gekennzeichnet, daß der Klebstoff für die Fixierung des hochsaugfähigen Materials (18) auf die Tragschicht (16) aufgesprüht wird, wobei die für das Aufsprühen verwendete Druckluft eine Restfeuchte von weniger als 0,5% hat.

4. Verfahren nach einem der vorhergehenden Ansprüche, dadurch gekennzeichnet, daß die Tragschicht (16) aus einem Tissuewerkstoff oder einem Vlieswerkstoff besteht.

5. Verfahren nach einem der vorhergehenden Ansprüche, dadurch gekennzeichnet, daß der hochsaugfähige Teil (18) des saugfähigen Materials an einer unteren Tragschicht (16) und auf dieser eine weitere Tragschicht (16') mit einem weiteren Teil (18') aus hochsaugfähigem Material angebracht wird.

6. Verfahren nach Anspruch 5, dadurch gekennzeichnet, daß mindestens das der unteren Tragschicht (16) zugeordnete hochsaugfähige Material (18) schichtförmig ausgebreitet wird und die Körner dieser Schicht (18) sich im wesentlichen in je einer Ebene erstrecken.

7. Verfahren nach einem der vorhergehenden Ansprüche, dadurch gekennzeichnet, daß die Klebstoffschicht sich über die Tragschicht (18) erstreckt.

8. Verfahren nach einem der vorhergehenden Ansprüche, dadurch gekennzeichnet, daß die auf der Tragschicht (16) aufgebrachte Acrylat-Granulat-Schicht (18) eine geringere Korngröße als die Acrylat-Granulat-Schicht (18') auf der weiteren Tragschicht (16') aufweist, welche Acrylat-Granulat-Schicht (18') auf der weiteren Tragschicht (16') insbesondere ebenfalls aufgeklebt wird.

9. Verfahren nach einem der vorhergehenden Ansprüche, dadurch gekennzeichnet, daß das hochsaugfähige Material auf der obersten Schicht (18') über eine an sich bekannte Deckschicht (20) aus Vlieswerkstoff, insbesondere aus einem Verbund von mindestens 20 % Viskose und höchstens 80% Propylen, oder Tissue abgedeckt wird.

10. Verfahren nach einem der vorhergehenden Ansprüche, dadurch gekennzeichnet, daß eine Abdeckbahn (26) aus Vlieswerkstoff mit einer hydrophoben Innenseite, insbesondere aus mindestens 80% Polypropylen und höchstens 20% Viskose, vorgesehen wird.

11. Verfahren nach einem der vorhergehenden Ansprüche, dadurch gekennzeichnet, daß die unterste Tragschicht (16) auf einer im wesentlichen feuchtigkeitsundurchlässigen Außenhaut (12), insbesondere aus Polypropylen, aufliegt oder durch diese Außenhaut gebildet wird.

## Claims

1. A method of producing an absorbent composite material, in particular for nappies mattress pads, sanitary towels, incontinence pads and liners and the like, having a support layer (16) on which a layer of absorbent material is applied, characterised in that at least one granular highly absorbent portion (18) of the absorbent material is adhered to the support layer (16) via an adhesive layer sprayed on using dried compressed air.

2. A method according to Claim 1, characterised in that the spray adhesive is applied externally to the highly absorbent material (18) which, in particular, consists of acrylate granules or which is biodegradable.

3. A method according to claim 1 or 2, characterised in that the adhesive for fixing the highly absorbent material (18) on the support layer (16) is sprayed on using compressed air having a residual moisture of less than 0.5 %.

4. A method according to any one of the preceding Claims, characterised in that the support layer (16) consists of a tissue material or a non-woven material.

5. A method according to any one of the preceding Claims, characterised in that the highly absorbent portion (18) of the absorbent material is applied to a lower support layer (16) and there is applied to this latter a further support layer (16') with a further portion (18') of highly absorbent material.

6. A method according to Claim 5, characterised in that at least the highly absorbent material (18) associated with the lower support layer (16) is spread in the form of a layer and the granules of this layer (18) each extend substantially in one plane.

7. A method according to one or more of the preceding Claims, characterised in that the adhesive layer extends over the support layer (18).

8. A method according to one or more of the preceding Claims, characterised in that the acrylate granule layer (18) applied to the support layer (16) has a smaller grain size than the acrylate granule layer (18') on the further support layer (16'), which acrylate granule layer (18') is in particular likewise adhered to the further support layer (16').

9. A method according to any one of the preceding Claims, characterised in that the highly absorbent material on the uppermost layer (18') is covered by way of a per se known cover layer (20) of non-woven material, in particular of a composite of at least 20 % viscose and at most 80 % propylene, or tissue.

10. A method according to any one of the preceding Claims, characterised in that a cover web (26) of non-woven material is provided with a hydrophobic inner side, in particular of at least 80 % polypropylene and at most 20 % viscose.

11. A method according to any one of the preceding Claims, characterised in that the lowermost support layer (16) is placed on a substantially moisture-impermeable outer skin (12), in particular of polypropylene, or is formed by this outer skin.

## Revendications

1. Procédé de fabrication d'un matériau composite absorbant, en particulier pour des couches, alèses, serviettes périodiques, garnitures pour incontinents et pour lits de malades ou similaires, avec une couche de support (16) sur laquelle est appliquée une couche de matériau absorbant, caractérisé en ce qu'au moins une partie granuleuse très absorbante (18) du matériau absorbant est collée sur la couche de support (16) par l'intermédiaire d'une couche de colle pulvérisée au moyen d'air comprimé séché.

2. Procédé selon la revendication 1, caractérisé en ce que la colle pulvérisée appliquée sur le matériau (18) très absorbant, composé en particulier de granulés d'acrylate ou biodégradable, est appliquée de l'extérieur.

3. Procédé selon la revendication 1 ou 2, caractérisé en ce que l'adhésif servant à la fixation du matériau très absorbant (18) sur la couche de support (16) est pulvérisé, l'air comprimé utilisé pour la pulvérisation contenant moins de 0,5 % d'humidité résiduelle.

4. Procédé selon l'une ou l'ensemble des revendications précédentes, caractérisé en ce que la couche de support (16) se compose de papier ouaté et d'un matériau non tissé.

5. Procédé selon l'une ou l'ensemble des revendications précédentes, caractérisé en ce que la partie très absorbante (18) du matériau absorbant est appliquée sur une couche de support (16) inférieure et une autre couche de support (16') pourvue d'une autre partie (18') de matériau très absorbant est posée sur celle-ci.

6. Procédé selon la revendication 5, caractérisé en ce qu'au moins le matériau très absorbant (18) associé à la couche de support (16) inférieure est étalé pour former une couche et les grains de cette couche (18) s'étendent sensiblement dans un plan.

7. Procédé selon l'une ou l'ensemble des revendications précédentes, caractérisé en ce que la couche d'adhésif s'étend par-dessus la couche de support (16).

8. Procédé selon l'une ou l'ensemble des revendications précédentes, caractérisé en ce que la couche de granulés d'acrylate (18) appliquée sur la couche de support (16) présente une grosseur de grains inférieure à la couche de granulés d'acrylate (18') posée sur l'autre couche de support (16'), laquelle couche de granulés d'acrylate (18') est également collée, en particulier, sur l'autre couche de support (16').

9. Procédé selon l'une ou l'ensemble des revendications précédentes, caractérisé en ce que le matériau très absorbant de la couche supérieure (18') est recouvert à l'aide d'une couche de couverture (20) connue en soi faite de matériau non tissé, en particulier d'un composite d'au minimum 20 % de viscose et au maximum 80 % de polypropylène, ou de papier ouaté.

10. Procédé selon l'une ou l'ensemble des revendications précédentes, caractérisé en ce qu'il est prévu une bande de couverture (26) faite d'un matériau non tissé à face interne hydrophobe, en particulier fait d'au minimum 80 % de polypropylène et au maximum 20 % de viscose.

11. Procédé selon l'une ou l'ensemble des revendications précédentes, caractérisé en ce que la couche de support inférieure (16) repose sur une enveloppe externe (12) sensiblement imperméable à l'humidité, faite en particulier de polypropylène, ou est formée de cette enveloppe externe.
